# EUROPÄISCHE PATENTSCHRIFT

(11) **EP 3 411 084 B1**
(45) Veröffentlichungstag und Bekanntmachung des Hinweises auf die Patenterteilung: **29.07.2026**
(21) Anmeldenummer: 17703676.1
(22) Anmeldetag: 27.01.2017
(51) Int. Cl.: A61L 2/07

(54) **FILTERSYSTEM MIT AUSLASSFUNKTION**
FILTER SYSTEM WITH OUTLET FUNCTION
SYSTÈME DE FILTRATION AVEC FONCTION DE SORTIE

(30) Priorität: 03.02.2016 DE 102016101912
(43) Veröffentlichungstag der Anmeldung: 12.12.2018
(73) Patentinhaber: Aesculap AG, 78532 Tuttlingen (DE)
(72) Erfinder: THOMAS, Stefan, 78532 Tuttlingen (DE); SAUTER, Wolfgang, 78603 Renquishausen (DE); TIMMERMANN, Jörg Hinrich, 78573 Wurmlingen (DE)
(74) Vertreter: Winter, Brandl - Partnerschaft mbB
(86) Internationale Anmeldenummer: PCT/EP2017/051725
(87) Internationale Veröffentlichungsnummer: WO 2017/133968

(56) Entgegenhaltungen:
- WO-A1-00/57930
- WO-A1-90/07346
- DE-A1- 3 202 430
- DE-A1- 4 111 077
- DE-B1- 1 642 161
- DE-C2- 4 111 077
- JP-A- 2011 206 619
- US-A- 4 580 719
- US-A- 5 352 416
- US-A1- 2013 280 134

## Beschreibung

Die vorliegende Erfindung betrifft ein gasdurchlässiges Filtersystem für einen Sterilbehälter, sowie einen Sterilbehälter mit einem Filtersystem.

Bei der überwiegenden Mehrheit klinischer Handlungen und Operationen ist die Gewährleistung der Sterilität der verwendeten Instrumente und / oder sonstiger Hilfsmittel unerlässlich. Aus diesem Grund werden Sterilcontainer /-behälter (auch als Sterilisationscontainer /-behälter bezeichnet) verwendet, welche beispielsweise mit medizinischen Instrumenten bestückt / befüllt werden. Daraufhin wird der Sterilbehälter im bestückten Zustand beispielsweise in einem Autoklaven für eine vorherbestimmte Zeitdauer auf eine vorherbestimmte Sterilisationstemperatur erhitzt, bis etwaige an den medizinischen Instrumenten anhaftende Mikroorganismen abgetötet sind.

Das Aufheizen des Behälters und des Inhalts mit Dampf bei der Dampfsterilisation führt zur Bildung von Kondensat innerhalb des Containers/Behälters, welches die Trocknungszeit des Sterilgutes verzögert oder im ungünstigsten Fall sogar zu feuchtem Sterilgut bei der Entnahme führt. Daher ist eine Ablassöffnung in dem Sterilbehälter vorteilhaft, da so das Kondensat direkt bei seiner Entstehung wieder abfließen kann und somit die Trocknung erleichtert. Zudem ist in dem Sterilbehälter ein Sterilfilter erforderlich, um nach dem Sterilisationsvorgang das Eindringen von Keimen in den Aufnahmeraum des Sterilbehälters zu verhindern. Es ist somit neben der Öffnung für den Gasaustausch eine zusätzliche Öffnung in der Behälterwandung notwendig, um das Kondensat auszuleiten.

Solche Sterilbehälter mit einem Sterilfilter und einer Ablauföffnung sind bereits aus dem Stand der Technik bekannt. Beispielsweise offenbart die DE 197 53 671 A1 einen Sterilbehälter, in dessen Deckel eine Filterscheibe angeordnet ist. Zusätzlich sind im Deckel und im Boden Öffnungen vorgesehen, welche von Dichtlippen abgedichtet werden. Überschreitet nun die Druckdifferenz zwischen dem Behälterinneren und dem Behälteräußeren einen bestimmten Wert, geben die Dichtlippen die Öffnungen frei und ein Fluid kann dem Sterilbehälter zu- oder abgeführt werden. Solch eine zusätzliche Öffnung oder auch nur eine zusätzliche Schnittstelle birgt ein zusätzliches Steril-Risiko. Außerdem muss diese Schnittstelle von Zeit zu Zeit zusätzlich neben anderen Komponenten der Sterilbarriere kontrolliert werden, was erhöhte Kosten und überdies mehr Arbeitsaufwand mit sich führt.

Weiterhin wird in der DE 20 2010 003 204 U1 ein Fluidauslass für einen Behälter beschrieben, welcher es ermöglicht, eine Öffnung in einem Behälterboden druckabhängig zu verschließen oder freizugeben. Dafür ist als Stellglied eine Druckkammer vorgesehen, die von einem zylindrischen Faltenbalg und zwei Deckeln ausgebildet wird. Einer der beiden Deckel ist fest mit einer Abdeckung verbunden und der andere ist so ausgebildet, dass er die Öffnung im Behälterboden vollständig verschließen kann, in der Ausgangsposition jedoch den Behälterboden nicht berührt. Wird jedoch der Druck in der Druckkammer größer als der Umgebungsdruck, dann streckt sich der Faltenbalg und drückt den Deckel gegen die Öffnung, sodass diese verschlossen wird. Eine ähnliche Konfiguration ist in der DE 41 11 077 C2 offenbart, jedoch wird hier die Öffnung im Behälterboden nicht in Abhängigkeit des Drucks, sondern der Temperatur verschlossen bzw. freigegeben.

Die WO 90/07346 A1 offenbart einen Sterilbehälter mit einem gasdurchlässigen Filtersystem, das einen sterilen Strömungspfad durch einen Filter in einen Innenraum des Sterilbehälters zulässt. Bei einer steigenden Temperatur erhöht sich ein Volumen in einem geschlossenen Balg und damit ein durch ihn ausgeübter Druck, der einer Vorspannung einer Feder entgegensteht. Übersteigt der Druck schließlich einen vorbestimmten Grenzwert, so wird ein unsteriler Strömungspfad in den Innenraum ausgebildet. Dieses Filtersystem ist sehr anfällig gegenüber Umgebungsbedingungen, da eine Höhe über dem Meeresspiegel bzw. der Luftdruck einen entscheidenden aber nicht ausgleichbaren Parameter darstellt und zudem das Gas in dem Balg nicht entweichen darf, um keinen Kalibrierungsfehler zu erzeugen. Damit ist das Filtersystem nicht dafür angepasst präzise bei Überschreiten einer vorbestimmten Temperatur eine Schaltung zwischen dem sterilen und unsterilen Pfad auszuführen, da die Temperatur einer Schaltung veränderlich ist und stark variiert.

Weitere Sterilbehälter mit einem Filtersystem offenbaren beispielsweise die JP 2011 206619 A, die US 5 352 416 A, die DE 32 02 430 A1, die DE 16 42 161 B1, die WO 00/57930 A1, die US 4 580 719 A sowie die US 2013/ 280134 A1.

Da die vorstehend beschriebenen Systeme die Auslassöffnung für das Kondensat immer entweder verschließt oder öffnet, ist es nicht möglich, in solch eine Auslassventilanordnung ein Filterelement zu integrieren. Es ist daher eine Aufgabe der vorliegenden Erfindung ein Filtersystem bereit zu stellen, welches über eine zusätzliche Auslassfunktion verfügt.

Diese Aufgabe wird durch ein Filtersystem und einen Sterilbehälter mit den Merkmalen der unabhängigen Ansprüche gelöst. Vorteilhafte Weiterbildungen der Erfindung sind in den jeweiligen Unteransprüchen angegeben.

Das erfindungsgemäße gasdurchlässige Filtersystem für einen Sterilbehälter enthält ein Filterelement, einen Dichtungsabschnitt, der ausgebildet ist, um an einem Sterilbehälterwandungsabschnitt des Sterilbehälters anliegend eine in dem Sterilbehälterwandungsabschnitt vorgesehene Gasaustauschöffnung dichtend zu

umschließen, sodass ein steriler Strömungspfad durch die Gasaustauschöffnung und das Filterelement ausgebildet wird, und mindestens einen temperatursensitiven Stellabschnitt, der bei Erreichen oder Überschreiten einer vorbestimmten Temperatur zumindest den Dichtungsabschnitt von dem Sterilbehälterwandungsabschnitt zwanghaft löst, sodass ein unsteriler Strömungspfad ausgebildet wird, welcher es erlaubt, ein Fluid unter Umgehung des Filterelements über die Gasaustauschöffnung zu- oder abzuführen.

Das Filtersystem besteht aus einem Filterelement, einem Dichtungsabschnitt und einem temperatursensitiven Stellabschnitt. Der Dichtungsabschnitt ist an der dem Sterilbehälterwandungsabschnitt, beispielsweise dem Sterilbehälterboden, zugewandten Seite des Filterelements und bevorzugt an dessen äußerem Rand angeordnet. Das Filterelement mit dem Dichtungsabschnitt kann an dem Sterilbehälterwandungsabschnitt anliegen und der temperatursensitive Stellabschnitt kann das Filterelement mit dem Dichtungsabschnitt von dem Sterilbehälterwandungsabschnitt lösen. Dabei ist die Temperatur des temperatursensitiven Stellabschnitts ausschlaggebend dafür, ob das Filterelement, genauer gesagt der Dichtungsabschnitt, an dem Sterilbehälterwandungsabschnitt anliegt oder nicht. Liegt die Temperatur des temperatursensitiven Stellabschnitts unterhalb der vorbestimmten Temperatur, d.h. unterhalb eines vorbestimmten Temperaturschwellwerts, kommt der Dichtungsabschnitt an dem Sterilbehälterwandungsabschnitt aufgrund einer Vorspannung des Filterelements, z.B: mittels eines Federelements, oder einer anderen, in Richtung des Sterilbehälterwandungsabschnitts wirkende Kraft zum anliegen. Erreicht oder überschreitet jedoch die Temperatur des temperatursensitiven Stellabschnitts die vorbestimmte Temperatur, so übt der temperatursensitive Stellabschnitt eine Kraft auf das Filterelement aus, welche dieses von dem Sterilbehälterwandungsabschnitt wegdrückt und damit ein zumindest teilweises, insbesondere vollständiges, Ablösen des Dichtungsabschnitts von dem Sterilbehälterwandungsabschnitt bewirkt.

Eine in dem Sterilbehälterwandungsabschnitt vorgesehene Gasaustauschöffnung kann in der Umfangsrichtung vollständig von dem Dichtungsabschnitt umschlossen werden, wenn der Dichtungsabschnitt an dem Sterilbehälterwandungsabschnitt anliegt. Dadurch wird sichergestellt, dass ein erster, steriler Strömungspfad durch das Filterelement und die Gasaustauschöffnung ausgebildet wird. Löst jedoch bei Erreichen oder Überschreiten der vorbestimmten Temperatur der temperatursensitive Stellabschnitt den Dichtungsabschnitt zumindest teilweise von dem Sterilbehälterwandungsabschnitt, dann wird zusätzlich ein zweiter, unsteriler Strömungspfad ausgebildet. Dieser zweite Strömungspfad ermöglicht es einem Fluid, z.B. Kondenswasser, durch die Gasaustauschöffnung zu strömen ohne zusätzlich das Filterelement passieren zu müssen.

Das erfindungsgemäße Filtersystem kombiniert somit eine Filterfunktion mit einer zusätzlichen Kondensatauslassfunktion. Es ist folglich möglich, Kondensat aus beispielsweise einem Sterilbehälter bereits während der Sterilisierzeit über eine sichere Ventillösung auszuleiten und es kann daher die Trocknungszeit von in dem Sterilbehälter befindlichen Sterilgut reduziert bzw. eine sichere Trocknung bei ungünstiger, schwerer Beladung sichergestellt werde. Da das Kondensat auf dem zweiten Strömungspfad das Filterelement nicht passieren muss, wird somit ein Durchnässen des Filterelements verhindert und deshalb kann ein ungehinderter Dampfaustausch im Sterilisationsprozess gewährleistet werden. Durch die Funktionskombination wird somit eine Lösung geschaffen, mit welcher die Trocknungszeit reduziert werden kann ohne die Sicherheit der Sterilbarriere zu beeinträchtigen.

Gemäß der Erfindung wird der temperatursensitive Stellabschnitt bei Erreichen oder Überschreiten der vorbestimmten Temperatur das Filterelement von einer ersten Position, in welcher das Filterelement parallel zu dem Sterilbehälterwandungsabschnitt ausgerichtet ist, in eine zweite Position, in welcher das Filterelement nicht parallel zu dem Sterilbehälterwandungsabschnitt ausgerichtet bzw. gekippt ist, bringen.

Gemäß einem Aspekt der Erfindung kann der Dichtungsabschnitt ein Dichtring sein.

Gemäß einem Aspekt der Erfindung kann der temperatursensitive Stellabschnitt mindestens ein von dem Filterelement separat ausgebildetes Stellelement sein.

Gemäß einem Aspekt der Erfindung kann der temperatursensitive Stellabschnitt mindestens eine von dem Filterelement separat ausgebildete Schnappscheibe sein.

Mit anderen Worten sind beispielsweise mehrere Schnappscheiben in dem Filtersystem angeordnet und derart mit dem Filterelement verbunden, dass sie eine Kraft auf dieses übertragen könne. Diese Schnappscheiben wechseln bei einer bestimmten Temperatur ihren Wölbungszustand, wodurch vorteilhafterweise auf einem recht kleinen Bauraum eine hohe Kompression bzw. Ausdehnung eines temperaturabhängigen Bauteils erzielt werden kann.

Gemäß einem Aspekt der Erfindung kann der temperatursensitive Stellabschnitt einstückig mit dem Filterelement ausgebildet sein.

Gemäß einem Aspekt der Erfindung kann der einstückig mit dem Filterelement ausgebildete temperatursensitive Stellabschnitt insgesamt als eine Schnappscheibe ausgebildet sein.

Durch diese Ausgestaltung wird die Anzahl der Bauteile des Filtersystems reduziert und die Kosten und die Komplexität des Zusammenbaus können verringert werden. Dies ist beispielsweise möglich, wenn eine Schnappscheibe mit integrierter Filterfunktion verwendet wird und der Dichtungsabschnitt direkt am äußeren Rand der dem Sterilbehälterwandungsabschnitt zugewandten Seite der Schnappscheibe angeordnet ist.

Gemäß einem Aspekt der Erfindung kann das Filterelement scheibenförmig ausgebildet sein.

Gemäß einem Aspekt der Erfindung kann das Filterelement durch mehrere gleichmäßig in der Umfangsrichtung verteilte, sich von einem Zentrum zu einem umlaufenden Rand erstreckende Stege verstärkt sein.

Gemäß einem Aspekt der Erfindung kann das Filterelement in einer Halterung eingefasst sein.

Gemäß einem Aspekt der Erfindung kann die Halterung durch mehrere gleichmäßig in der Umfangsrichtung verteilte, sich von einem Zentrum zu einem umlaufenden Rand erstreckende Stege verstärkt sein.

Idealerweise stützt sich das Filterelement nur an seinem äußeren Randabschnitt an dem Dichtungsabschnitt ab. Dadurch wird die von dem Dichtungsabschnitt umschlossene Gasaustauschöffnung des Sterilbehälterwandungsabschnitts durch keine Stützstrukturen verdeckt und ein hoher Fluiddurchsatz durch die Öffnung kann gewährleistet werden. Dafür ist es jedoch notwendig, dass das Filterelement in seiner radialen Richtung verstärkt ist. Dies kann einerseits durch speichenartig angeordnete Verstärkungsstege, die in das Filterelement integriert oder auf diesem aufgebracht sind, realisiert werden. Andererseits kann das Filterelement in einer Halterung aufgenommen sein und somit eine Filterkassette ausbilden. In diesem Fall ist es möglich die Halterung auf die vorstehend beschriebene Art und Weise zu verstärken. Falls eine Schnappscheibe mit integrierter Filterfunktion verwendet wird, ist es außerdem denkbar, dass diese Schnappscheibe verstärkt ist.

Der erfindungsgemäße Sterilbehälter, insbesondere zur Aufnahme und sterilen Aufbewahrung von chirurgischen Besteck oder chirurgischen Material, enthält einen durch einen Sterilbehälterboden und Sterilbehälterwänden gebildeten Aufnahmeraum und einem Sterilbehälterdeckel zum Verschließen des Aufnahmeraums, wobei mindestens ein erfindungsgemäßes Filtersystem an einem Sterilbehälterwandungsabschnitt befestigt ist, welcher eine Gasaustauschöffnung aufweist.

Gemäß einem Aspekt der Erfindung ist das mindestes eine Filtersystem an dem Behälterboden befestigt.

Dadurch, dass das Filtersystem in dem Sterilbehälter vorgesehen ist, können auf die vorstehend beschriebene Art und Weise zwei Strömungspfade von dem Behälterinneren zu dem Behälteräußeren oder umgekehrt ausgebildet werden. Dadurch können mit nur einer Öffnung in einem Sterilbehälterwandungsabschnitt, z.B. dem Sterilbehälterboden, eine Filterfunktion und eine Kondensatauslassfunktion realisiert werden. Da das Filtersystem um eine Funktion erweitert wird, jedoch gegenüber seinem standardmäßigem Aufbau größtenteils gleichbleibt, ist es möglich, die bisher im Sterilbehälterdeckel platzierten Filtersysteme in den Sterilbehälterboden des Sterilbehälters zu adaptieren und ihr Funktionsweise um die Kondensatauslassfunktion zu erweitern. Es können folglich in dem erfindungsgemäßen Sterilbehälter ähnliche Bauteile wie in bekannten Behältern mit lediglich einem konventionellen Filtersystem verwendet werden, was dieselben Herstell- und Montageprozesse ermöglicht und somit die Herstellkosten nur geringfügig erhöht bzw. gegenüber Sterilbehältern mit separatem Filtersystem und Kondensatauslassöffnung reduziert. Aufgrund der Anlehnung an bekannte Lösungen bleiben die Risiken vergleichbar gering und das Systemgewicht, das Beladegewicht, das nutzbare Volumen und der Preis im Vergleich zu derzeitigen Standardbehältern mit lediglich einer Filterfunktion bleiben im Wesentlichen unverändert.

Mit anderen Worten wird die Öffnungsfunktion des Filtersystems bei Unterdruck im Sterilbehälter erweitert, sodass das Filterelement mit Dichtungsabschnitt zusätzlich prozessgesteuert in Abhängigkeit der Temperatur von dem Sterilbehälterboden abgehoben werden kann, um einen gezielten Kondensatauslass zu ermöglichen, was die Trocknungszeit erheblich reduziert. Der erfindungsgemäße Sterilbehälter hat somit die gleiche Anzahl von Schnitt-, Dicht- und Problemstellen und kann somit in gewohnter Art und Weise als sicher angesehen werden. Zusätzlicher Montage- und Kontrollaufwand wird durch diese Kombi-Lösung von Filter- und Auslassfunktion nicht generiert. Infolgedessen kann auf ein Filtersystem im Sterilbehälterdeckel verzichtet werden.

Gemäß einem Aspekt der Erfindung kann der temperatursensitive Stellabschnitt bei Erreichen oder Überschreiten der vorbestimmten Temperatur das Filterelement von einer ersten Position, in welcher das Filterelement parallel zu dem Sterilbehälterboden ausgerichtet ist, in eine zweite Position, in welcher das Filterelement nicht parallel zu dem Sterilbehälterboden ausgerichtet bzw. gekippt ist, bringen. Alternativ kann der Stellabschnitt dergestalt sein, dass das Filterelement in der zweiten Position auch parallel zu dem Sterilbehälterboden ausgerichtet ist.

Wenn beispielsweise der temperatursensitive Stellabschnitt als ein separates Stellelement in Form einer Schnappscheibe oder eines Schnappscheibenpakets ausgebildet ist, kann das Stellelement das Filterelement nur an einer Stelle berühren bzw. die Kraft nur an einer Stelle einleiten. Dadurch wird bei einer Expansion des Stellelements das Filterelement gegenüber dem Sterilbehälterboden derart angehoben und geneigt, dass nur ein Teil des Dichtungsabschnitts von dem Sterilbehälterboden gelöst wird. Im Umkehrschluss bedeutet dies, dass ein Teil des Dichtungsabschnitts permanent mit dem Sterilbehälterboden in Kontakt bleibt und eine zuverlässige Dichtung ausbildet.

Gemäß einem Aspekt der Erfindung kann das Filtersystem ferner eine Abdeckung und eine Feder aufweisen.

Gemäß einem Aspekt der Erfindung kann das Filtersystem über die Abdeckung an dem Sterilbehälterboden arretiert werden.

Gemäß einem Aspekt der Erfindung kann die Abdeckung mittels Bajonettverschluss an dem Sterilbehälterboden befestigt werden.

Gemäß einem Aspekt der Erfindung kann die Abdeckung in eine in dem Sterilbehälterboden ausgebildete Aufnahme eingeclipst werden.

Durch die vorstehend genannten Befestigungsmethoden ist es möglich die Abdeckung und somit das Filtersystem ohne zusätzliche Hilfsmittel oder Bauteile, einfach und schnell und somit auch günstig in dem Sterilbehälter zu befestigen.

Gemäß einem Aspekt der Erfindung kann die Feder als eine Art Blatt- oder Tellerfeder ausgebildet sein.

Gemäß einem Aspekt der Erfindung kann sich die Feder an der Abdeckung und an dem Filterelement abstützen.

Gemäß einem Aspekt der Erfindung kann sich die Feder in der Mitte der Abdeckung und an dem Filterelement abstützen.

Gemäß einem Aspekt der Erfindung kann die Feder an der Abdeckung befestigt sein.

Die Feder ist somit zwischen der Abdeckung und dem Filterelement angeordnet und ihre Vorspannkraft drückt das Filterelement von der Abdeckung in Richtung des Sterilbehälterwandungsabschnitts weg. Dadurch dass die Feder als eine Art Blatt- oder Tellerfeder ausgebildet ist, ist es zudem möglich, ihre Vorspannkraft möglichst im äußeren Randbereich, d.h. in dem Bereich, in dem der Dichtungsabschnitt vorgesehen ist, auf das Filterelement zu übertragen. Ergo wird der Dichtungsabschnitt maximal an den Sterilbehälterwandungsabschnitt gedrückt und somit wird eine zuverlässige Dichtung ausgebildet.

Gemäß einem Aspekt der Erfindung kann die Feder eine Vorspannkraft entgegen der durch den temperatursensitiven Stellabschnitt aufgebrachten Kraft ausüben.

Gemäß einem Aspekt der Erfindung kann die Vorspannkraft der Feder kleiner als die durch den temperatursensitiven Stellabschnitt aufgebrachte Kraft sein, wenn die vorbestimmte Temperatur erreicht oder überschritten wird, und kann die Vorspannkraft der Feder größer als die durch den temperatursensitiven Stellabschnitt aufgebrachte Kraft sein, wenn die vorbestimmte Temperatur nicht erreicht oder überschritten wird.

Folglich wird unterhalb einer vorbestimmten Temperatur der Dichtungsabschnitt aufgrund der Vorspannkraft gegen den Sterilbehälterboden gedrückt. Wird diese vorbestimmte Temperatur jedoch erreicht oder überschritten, ändert der temperatursensitive Stellabschnitt seine Ausdehnung, beispielsweise wird die Wölbungsrichtung einer Schnappscheibe geändert, was die von ihm auf das Filterelement ausgeübte Kraft erhöht. Infolgedessen wird das Filterelement mit Dichtungsabschnitt entgegen der Vorspannkraft der Feder von dem Sterilbehälterboden weggeschoben und die durch den Dichtungsabschnitt ausgebildete Dichtung wird zwanghaft gelöst.

Gemäß einem Aspekt der Erfindung kann in der Abdeckung mindestens eine seitliche Öffnung vorgesehen ist.

Gemäß einem Aspekt der Erfindung kann die seitlich Öffnung auf der dem Sterilbehälterboden zugewandten Seite der Abdeckung vorgesehen sein, sodass ein in dem Aufnahmeraum des Sterilbehälters befindliches Fluid durch die seitliche Öffnung und die Gasaustauschöffnung aus dem Sterilbehälter abgeführt werden kann.

Gemäß einem Aspekt der Erfindung ist die Abdeckung als ein Blechteil ausgeführt.

Die Abdeckung umschließt das Filterelement vollständig, sodass das ein Übergießen des Filterelements verhindert werden kann. Lediglich über die seitliche Öffnung kann das Kondensat in das Innere der Abdeckung gelangen, und sich auf dem Sterilbehälterboden sammeln. Der Dichtungsabschnitt ist dabei so dimensioniert, das er einen ausreichenden Sicherheitsabstand zwischen dem Filterelement und dem Sterilbehälterboden gewährleistet und somit ein Fluten des Filterelements verhindert.

Gemäß einem Aspekt der Erfindung können in dem Sterilbehälterboden mehrere Öffnungen, welche die Gasaustauschöffnung ausbilden, vorgesehen sein.

Gemäß einem Aspekt der Erfindung können diese Öffnungen in einer Kreisform angeordnet sein.

Gemäß einem Aspekt der Erfindung kann ein Kreissegment in dem Sterilbehälterboden, an welchem sich der temperatursensitive Stellabschnitt abstützt, keine Öffnungen aufweisen.

Dadurch dass der Abstützbereich im Sterilbehälterboden nicht durch Öffnungen geschwächt ist, kann ein optimale Krafteinleitung von dem temperatursensitiven Stellabschnitt über den Abstützbereich in den Sterilbehälter gewährleistet werden.

### Kurze Beschreibung der Zeichnungen

Fig. 1 zeigt eine Querschnittsansicht eines an einem Sterilbehälterboden befestigtes Filtersystems gemäß einer ersten Ausführungsform der Erfindung in einem kalten Zustand.
Fig. 2 zeigt eine Querschnittsansicht des an dem Sterilbehälterboden befestigten Filtersystems gemäß der ersten Ausführungsform der Erfindung in einem warmen Zustand.
Fig. 3 zeigt eine perspektivische Ansicht eines Filtersystems gemäß einer zweiten Ausführungsform der Erfindung in Form einer Kombi-Schnappscheibe.
Fig. 4 zeigt die Kombi-Schnappscheibe in einem kalten Zustand.
Fig. 5 zeigt die Kombi-Schnappscheibe in einem warmen Zustand.
Fig. 6 zeigt eine perspektivische Ansicht eines Sterilbehälters mit darin angeordneten Filtersystemen.
Fig. 7 zeigt eine perspektivische Ansicht des Sterilbehälters ohne Filtersysteme.

### Detaillierte Beschreibung bevorzugter Ausführungsformen

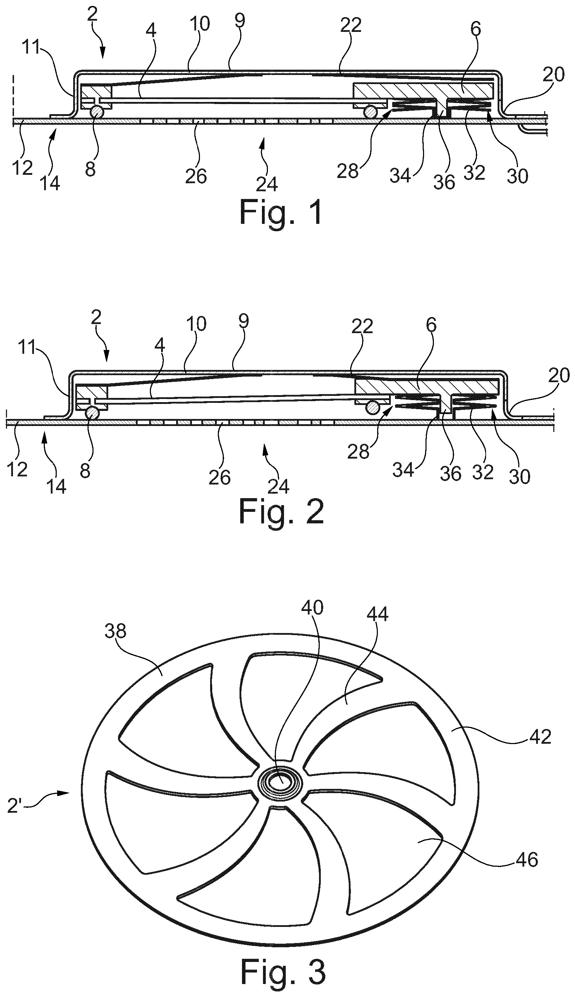

Nachfolgend wird mit Bezug auf die Fign. 1 und 2 eine erste Ausführungsform eines erfindungsgemäßen Filtersystems 2 beschrieben.

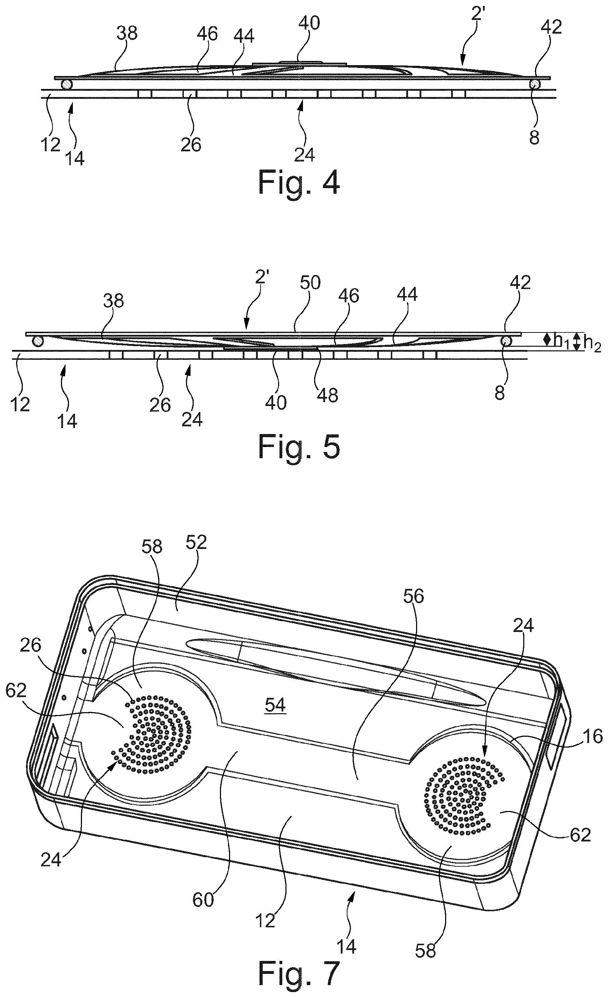

Fig. 1 zeigt das Filtersystem 2 in einem kalten Zustand. Das Filtersystem 2 umfasst ein scheibenförmiges Filterelement 4, welches in einer ringförmigen Halterung 6 fest eingefasst bzw. eingepresst ist, und einen Dichtring 8. Der Dichtring 8 ist in einem äußeren Bereich der Halterung 6 angeordnet, welcher dem Außendurchmesser des Filterelements 4 entspricht. Mit anderen Worten entspricht der Durchmesser des Dichtrings 8 in etwa dem Durchmesser des Filterelements 4. Die Halterung 6 mit Filterelement 4 und Dichtring 8 ist in einer topfförmigen Abdeckung 10 aufgenommen. Von einem kreisförmigen Deckelabschnitt 9 der Abdeckung 10 erstreckt sich eine umlaufende Umfangswandung 11. Die Abdeckung 10 kann z.B. durch Aufclipsen, Aufschrauben oder Drehen, etc an einem Sterilbehälterboden 12 eines Sterilbehälters 14 befestigt werden. Dafür ist in dem Sterilbehälterboden 12 eine (in Fig. 7 gezeigte) Aussparung 16 vorgesehen, in welche ein sich an die Umfangswandung 11 anschließender Flansch 18 der Abdeckung 10 eingreift. Außerdem sind in der Umfangswandung 11 der Abdeckung 10 mehrere in Umfangsrichtung verteilte seitliche oder radiale Öffnungen 20 vorgesehen, welche einen Fluidaustausch aus der Abdeckung 10 heraus oder in die Abdeckung 10 hinein erlauben. Die seitlichen Öffnungen 20 sind auf der dem Sterilbehälterboden 12 zugewandten Seite der Umfangswandung 11 angeordnet und bilden Unterbrechungen des Flansches 18.

Weiterhin beinhaltet das Filtersystem 2 eine als Tellerfeder ausgebildete Feder 22, welche zwischen der Abdeckung 10 und der Halterung 6 angeordnet und an der Abdeckung 10 befestigt ist. Dabei stützt sich die Feder 22 in der Mitte der Abdeckung 10 und an einem äußeren Rand der Halterung 6 ab. Aufgrund der Vorspannkraft der Feder 22 wird somit die Halterung 6 und damit der Dichtring 8 gegen den Sterilbehälterboden 12 gedrückt, wenn die Abdeckung 10 an dem Sterilbehälterboden 12 befestigt ist. In dem Sterilbehälterboden 12 ist eine Gasaustauschöffnung 24 mit mehreren kreisförmigen Auslassöffnungen 26 vorgesehen. Dabei sind die Gasaustauschöffnung 24 und das Filtersystem 2 so zueinander positioniert, dass der Dichtring 8 die Gasaustauschöffnung 24 in der Umfangsrichtung vollständig umschließt, wenn der Dichtring 8 an dem Sterilbehälterboden 12 anliegt. Dadurch wird ein steriler Strömungspfad von dem Behälteräußeren durch die Gasaustauschöffnung 24, das Filterelement 4 und die seitliche Öffnung 20 in das Behälterinnere ausgebildet.

Die Montage des Filtersystems 2 erfolgt dabei in nur zwei einfachen Schritten. Zuerst wird das Filterelement 4 mit Dichtring 8 über die Gasaustauschöffnung 24 des Sterilbehälterbodens 12 gesetzt. Anschließend wird die Abdeckung 10 an dem Sterilbehälterboden befestigt. Dabei wird das Filterelement 4 über die in der Abdeckung 10 befestigte Feder 22 in seiner Position fixiert. Hierbei entsteht keine feste Verbindung zwischen der Feder 22 und dem Filterelement 4 bzw. der Halterung 6, was die Montage vereinfacht. Mit anderen Worten gleiten die Feder 22 und die Halterung 6 aneinander ab und die Feder 22 wird beim Arretieren der Abdeckung 10 durch die Abdeckung 10 und die Halterung 6 vorgespannt. Diese Tatsache ist in Bezug auf die Prozesssicherheit von besonderem Vorteil, da bei einer Montage oder einer Wiedermontage kein Bauteil vergessen werden kann. Der Grund dafür ist die haptische Rückmeldung, welche der Anwender erfährt, wenn das System vollständig montiert wurde. Ohne die Abdeckung 10 kann die Halterung 6 mit dem Filterelement 4 nicht arretiert werden und ohne die Halterung 6 mit dem Filterelement 4 wird die Feder 22 nicht vorgespannt und der Anwender erfährt keine haptische Rückmeldung beim Arretieren der Abdeckung 10. Mit anderen Worten wird ein Fehler beim Zusammenbau erkannt, wenn die Abdeckung 10 nicht entgegen der Vorspannkraft der Feder 22 gegen den Sterilbehälterboden 12 gedrückt werden muss.

Ferner umfasst das Filtersystem 2 eine ebenfalls in der Abdeckung 10 angeordnete temperatursensitive Stelleinheit 28. Dieser umfasst ein Schnappscheibenpaket 30 mit mehreren, in dieser Ausführungsform vier, Schnappscheiben 32 und einer Hülse 34. Die Stelleinheit 28 ist dabei zwischen der Halterung 6 und dem Sterilbehälterboden 12 derart angeordnet, dass sich das eine Ende des Schnappscheibenpakets 30 an der Halterung 6 abstützt oder an ihr befestigt ist und das andere Ende des Schnappscheibenpakets 30 mit der Hülse 34 verbunden ist, welche wiederum auf dem Sterilbehälterboden 12 aufliegt. An der Halterung 6 ist ferner auf der dem Sterilbehälterboden 12 zugewandeten Seite ein zylinderförmiger Vorsprung 36 ausgebildet, welcher in die Hülse 34 hineinragt und mit Spiel in der Hülse 34 geführt wird.

Die Schnappscheiben 32 sind aus einem Formgedächtnismaterial wie beispielsweise einem Bimetall ausgebildet. Dies bewirkt, dass die gewölbten Schnappscheiben 32 in Abhängigkeit ihrer Temperatur ihre Wölbungsrichtung wechseln können. Dadurch ist es möglich, die Schnappscheiben 32 so in dem Schnappscheibenpaket 30 anzuordnen, dass sich das Schnappscheibenpaket 30 unterhalb einer vorbestimmten Temperatur, der Schnapptemperatur, in einem ersten, komprimierten Zustand befindet. Bei Erreichen oder Überschreiten der Schnapptemperatur wechseln die Schnappscheiben 32 ihre Wölbungsrichtung, was dazu führt, dass sie sich nun voneinander abstoßen und sich das Schnappscheibenpaket 30 in einem zweiten, ausgedehnten Zustand befindet, welcher in Fig. 2 dargestellt ist. Mit anderen Worten ist die Höhe des Schnappscheibenpakets 30 im zweiten Zustand größer als die im ersten Zustand.

Wenn somit, wie in Fig. 2 gezeigt, das Filtersystem 2 in einem heißen Zustand ist, d.h. die Temperatur der Schnappscheiben 32 die Schnapptemperatur erreicht oder überschreitet, dann dehnt sich das Schnappscheibenpaket 30 aus und drückt über die Hülse 34 die Halterung 6 entgegen der Vorspannkraft der Feder 22 von dem Sterilbehälterboden 12 weg. Mit anderen Worten ist die durch die Schnappeinheit 28 auf die Halterung 6 ausgeübte Kraft größer als die Vorspannkraft der Feder 22, wenn die Temperatur der Schnappscheiben 32 die Schnapptemperatur erreicht oder überschreitet. Andernfalls ist die Vorspannkraft der Feder 22 größer und die Halterung 6 bzw. der Dichtring 8 wird gegen den Sterilbehälterboden 12 gedrückt. In dem Fall, in dem sich das Schnappscheibenpaket 30 in dem ausgedehnten Zustand befindet, d.h. es die Halterung 6 von dem Sterilbehälterboden 12 wegdrückt, wird somit der Dichtring 8 zumindest teilweise von dem Sterilbehälterboden 12 gelöst. Dadurch wird ein zweiter, unsteriler Strömungspfad ausgebildet, welcher von dem Behälteräußeren über die Gasaustauschöffnung 24 und die seitliche Öffnung 20 in das Behälterinnere führt. Auf diesem zweiten Strömungspfad muss ein Fluid somit nicht das Filterelement 4 durchströmen.

Wird also bei einem Sterilisationsprozess, beispielsweise mit gesättigtem Wasserdampf, die Schnapptemperatur erreicht oder überschritten, dehnt sich das Schnappscheibenpaket 30 aus und gibt somit den zweiten Strömungspfad, welcher die Umgehung des Filterelements ermöglicht, frei. Dadurch kann zum Einen der gesättigte Wasserdampf schneller in den Sterilbehälter 14 einströmen, was notwendig ist, um eine Beschädigung des Sterilbehälters14 aufgrund der Druckdifferenz zwischen dem Behälterinneren und dem Behälteräußeren zu verhindern. Zum Anderen kann so gleich das sich während des Sterilisationsprozesses im Sterilbehälter 14 bildende Kondensat abfließen. Gerade bei einem voll beladenen Sterilbehälter 14 bildet sich aufgrund der großen, kalten Masse im Behälterinneren viel Kondensat, welches ohne die zusätzliche Auslassfunktion des Filtersystems 2 die Trocknungszeit des Sterilguts erheblich verlängern würde.

Fällt die Temperatur der Schnappscheiben 32 wieder unter die Schnapptemperatur, wechseln diese ihren Wölbungszustand und das Schnappscheibenpaket 30 wird komprimiert. Dadurch wird die Halterung 6 nicht mehr durch das Schnappscheibenpaket 30 von dem Sterilbehälterboden 12 weggedrückt. Vielmehr bewirkt die Vorspannkraft der Feder 22, dass sich die Halterung 6 in Richtung des Sterilbehälterbodens 12 bewegt, sodass der Dichtring 8 die Gasaustauschöffnung 24 wieder dichtend umschließt. Das Filtersystem 2 kehrt somit in seinen in Fig. 1 dargestellten kalten Ausgangzustand zurück. In diesem Zustand ist wie vorstehend beschrieben wurde lediglich der sterile Strömungspfad durch das Filterelement 4 freigegeben. Die restliche im Behälterinneren verbleibende Feuchtigkeit muss somit über die Luft und durch das Filterelement 4 abgeführt werden.

In der vorstehend beschriebenen Konfiguration sind der Dichtring 8 und die Halterung 6 so dimensioniert, dass immer ein Mindestabstand zwischen dem Filterelement 4 und dem Sterilbehälterboden 12 ausgebildet ist, der größer ist als der maximal erwartete Wasserstand im Sterilbehälter 14. Dadurch wird ein Durchnässen des Filterelements 4 zuverlässig verhindert. Weiterhin ist in der vorstehend beschriebenen Konfiguration lediglich eine temperatursensitive Stelleinheit 28 vorgesehen. Dies bewirkt ein Verkippen der Halterung 6 und des Filterelements 4 gegenüber dem Sterilbehälterboden 12. Dadurch bleibt auch immer ein Teil des Dichtrings 8 mit dem Sterilbehälterboden 12 in Kontakt. Es wäre jedoch auch möglich, zwei oder mehr temperatursensitive Stelleinheiten 28 in dem Filtersystem 2 vorzusehen und somit ein gleichmäßiges Abheben der Halterung 6 mit dem Filterelement 4 und dem Dichtring 8 von dem Sterilbehälterboden 12 zu gewährleisten.

Weiterhin sei erwähnt, dass das Filterelement 4 sehr einfach ersetzt oder beidseitig kontrolliert werden kann, da lediglich die Abdeckung 10 entfernt werden muss. Zusätzlich ist eine Reinigung so sehr einfach möglich. Außerdem ist das gesamte Filtersystem 2 sehr einfach aufgebaut, da es im Wesentlichen nur aus drei Teilen besteht, der Halterung 6 mit dem Filterelement 4, der Stelleinheit 28 und der Abdeckung 10 mit der darin befestigten Feder 22. Dadurch liegen zu keinem Zeitpunkt Kleinteile oder einzelne Bauteile vor, was das Handling vereinfacht. Zudem wird aufgrund der geringen Anzahl der Komponenten und der genutzten Leichtbaukonzepte das Gesamtgewicht des Filtersystems reduziert.

Nachfolgend wird mit Bezug auf die Fign. 3 bis 5 eine zweite Ausführungsform eines erfindungsgemäßen Filtersystems 2' beschrieben. In der zweiten Ausführungsform werden das Filterelement 4, die Halterung 6 und die Stelleinheit 28 durch die in Fig. 3 gezeigte Kombi-Schnappscheibe 38 ersetzt. Der Anordnung der restlichen Bauteile bleibt gegenüber der ersten Ausführungsform unverändert.

Bei der in Fig. 3 gezeigten Kombi-Schnappscheibe 38 erstrecken sich von einem Zentrum 40 zu einem umlaufenden Rand 42 mehrere in der Umfangsrichtung gleichmäßig verteilte Stege 44. In den von dem Zentrum 40, dem Rand 42 und den Stegen 44 ausgebildeten Flächen ist ein Filtermaterial 46 vorgesehen. Somit vereint die Kombi-Schnappscheibe 38 die Funktionen einer Schnappscheibe und eines Filters. Es ist jedoch auch möglich, die Kombi-Schnappscheibe 38 aus einer unteren Klemmscheibe und einer oberen Klemmscheibe auszubilden und das Filterelement 46 fest und somit dicht zwischen den beiden Klemmscheibe einzupressen. Alternativ können auch das Filterelement 4 und/oder die Halterung 6 der ersten Ausführungsform auf die vorstehend beschriebene Art und Weise mit Stegen verstärkt sein.

Mit Bezug auf die Fign. 4 und 5 wird die Funktionsweise der zweiten Ausführungsform beschrieben. In Fig. 4 befindet sich die Kombi-Schnappscheibe 38 in ihrem kalten Zustand. D.h. ihre Temperatur ist unterhalb der Schnapptemperatur und die Kombi-Schnappscheibe 38 ist nach oben bzw. von dem Sterilbehälterboden 12 weg gewölbt. Auf der dem Sterilbehälterboden 12 zugewandten Seite der Kombi-Schnappscheibe 38 ist am umlaufenden Rand 42 der Dichtring 8 angeordnet. Aufgrund der (in Fig. 4 nicht dargestellten Feder 22) kommt der Dichtring 8 an dem Sterilbehälterboden 12 zum Anliegen und umschließt die Gasaustauschöffnung 24 dichtend. Es wird somit lediglich ein steriler Strömungspfad durch die Gasaustauschöffnung 24 und das Filtermaterial 46 ausgebildet.

Erreicht oder überschreitet nun die Temperatur der Kombi-Schnappscheibe 38 die Schnapptemperatur, kommt die Kombi-Schnappscheibe 38 in ihren warmen Zustand und ihre Wölbung kehrt sich um (siehe Fig. 5). Sie ist nun nach unten bzw. in Richtung des Sterilbehälterbodens 12 gewölbt. Dabei kommt ein am Zentrum 40 der Kombi-Schnappscheibe 38 ausgebildeter Zentrumsvorsprung 48 mit dem Sterilbehälterboden 12 in Kontakt. Da eine Höhe h1 von einer Grundseite 50 der Kombi-Schnappscheibe 38 zu der Unterseite des Dichtrings 8 kleiner ist als eine Höhe h2 von der Grundseite 50 zu dem Zentrumsvorsprung 48, hebt sich der Dichtring 8 von dem Sterilbehälterboden 12 ab und ein weiterer Strömungspfad wird ausgebildet, welcher die Umgehung des Filtermaterials 46 erlaubt. Das Abheben des Dichtrings 8 von dem Sterilbehälterboden 12 ist außerdem möglich, da die Spannkraft der Kombi-Schnappscheine 38 größer ist als die Vorspannkraft der Feder 22. Die Folgen und Vorteile des zusätzlichen Strömungspfades entsprechen im Wesentlichen denen der ersten Ausführungsform.

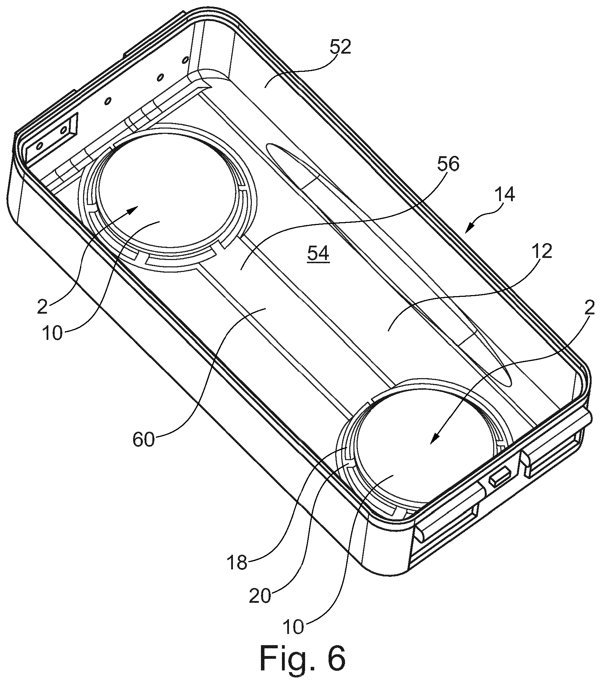

In den Fign. 6 und 7 ist ein erfindungsgemäßer Sterilbehälter 14 gezeigt. Dabei bilden mehrere Sterilbehälterseitenwände 52 und der Sterilbehälterboden 12 einen Aufnahmeraum 54 für medizinische Instrumente aus, welcher durch einen nicht dargestellten Sterilbehälterdeckel verschlossen werden kann. In der in Fig. 6 gezeigten Konfiguration sind zwei Filtersysteme 2 an dem Sterilbehälterboden 12 befestigt. Es ist jedoch auch möglich nur eines oder drei oder mehr Filtersysteme 2 in dem Sterilbehälter 14 vorzusehen. Alternativ können die Filtersysteme 2 auch an einer Sterilbehälterseitenwand 52 oder dem Sterilbehälterdeckel angeordnet sein.

Fig. 7 zeigt den Sterilbehälter 14 ohne Filtersysteme 2. In dem Sterilbehälterboden 12 ist eine Vertiefung 56 vorgesehen. Diese Vertiefung 56 erstreckt sich über den Bereich zweier Aufnahmen 58 für die Filtersysteme 2 und einen Kanal 60, der zwischen den beiden Aufnahmen 58 vorgesehen ist. In der Vertiefung 56 kann sich das Kondenswasser sammeln, bevor es durch die Auslassöffnungen 26 der Gasaustauschöffnung 24 aus dem Sterilbehälter 14 austritt. Zudem ist am Rand der Vertiefung 56 im Bereich der Aufnahme 58 die Aussparung 16 angeordnet, über welche die Abdeckung 10 mit ihren Flanschen 18 an dem Sterilbehälterboden 12 befestigt wird. Jedoch kann die Abdeckung 10 auch auf eine andere Art und Weise an dem Sterilbehälterboden 12 befestigt sein. Es ist beispielsweise auch möglich die Abdeckung 10 mittels Bajonettverschluss am dem Sterilbehälterboden 12 zu arretieren.

Weiterhin ist in Fig. 7 die Anordnung der Auslassöffnungen 26 der Gasaustauschöffnung 24 im Sterilbehälterboden 12 für die erste Ausführungsform gezeigt. Dabei sind die Auslassöffnungen 26 in sechs konzentrischen Kreisen angeordnet, wobei ein Kreissegment keine Auslassöffnungen 26 aufweist. In diesem Abstützbereich 62 ist die Stelleinheit 28 des Filtersystems 2 angeordnet. Genauer gesagt stützt sich in diesem Abstützbereich 62 die Hülse 34 der Stelleinheit 28 an dem Sterilbehälterboden 12 ab, weshalb es aus Gründen der Stabilität und der Krafteinleitung vorteilhaft ist, den Sterilbehälterboden 12 in dem Abstützbereich 62 nicht durch Öffnungen zu schwächen. Im Falle der zweiten Ausführungsform ist es auch möglich, den Abstützbereich 62 im Zentrum der Gasaustauschöffnung 24 vorzusehen, sodass sich der Zentrumsvorsprung 48 der Kombi-Schnappscheibe 38 in dem Abstützbereich 62 abstützen kann. Die Auslassöffnungen 26 können in diesem Fall konzentrisch um den Abstützabschnitt 62 angeordnet sein.

Neben den vorstehend beschriebenen Ausführungsformen sind auch noch alternative Ausgestaltungen des Filtersystems 2 und des Sterilbehälters 14 möglich. So kann beispielsweise die Feder 22 am äußeren Rand der Halterung 6 befestigt sein und sich an der der Halterung 6 zugewandeten Seite der Abdeckung 10 abstützen.

## Patentansprüche

1. Gasdurchlässiges Filtersystem (2; 2') für einen Sterilbehälter (14), mit einem Filterelement (4; 46), einen Dichtungsabschnitt (8), der ausgebildet ist, um an einem Sterilbehälterwandungsabschnitt (12) des Sterilbehälters (14) anliegend eine in dem Sterilbehälterwandungsabschnitt (12) vorgesehene Gasaustauschöffnung (24) dichtend zu umschließen, sodass ein steriler Strömungspfad durch die Gasaustauschöffnung (24) und das Filterelement (4; 46) ausgebildet wird, und mindestens einem temperatursensitiven Stellabschnitt (28; 38), der bei Erreichen oder Überschreiten einer vorbestimmten Temperatur zumindest den Dichtungsabschnitt (8) von dem Sterilbehälterwandungsabschnitt (12) zwanghaft löst, sodass ein unsteriler Strömungspfad ausgebildet wird, welcher es erlaubt, ein Fluid unter Umgehung des Filterelements (4; 46) über die Gasaustauschöffnung (24) zu- oder abzuführen, **dadurch gekennzeichnet, dass** der temperatursensitive Stellabschnitt (28; 38) bei Erreichen oder Überschreiten der vorbestimmten Temperatur das Filterelement (4; 46) von einer ersten Position, in welcher das Filterelement (4; 46) parallel zu dem Sterilbehälterwandungsabschnitt (12) des Sterilbehälters (14) ausgerichtet ist, in eine zweite Position, in welcher das Filterelement (4; 46) nicht parallel zu dem Sterilbehälterwandungsabschnitt (12) ausgerichtet ist, bringt.

2. Filtersystem (2) gemäß Anspruch 1, **dadurch gekennzeichnet, dass** der temperatursensitive Stellabschnitt (28) mindestens ein von dem Filterelement (4) separat ausgebildetes Stellelement ist.

3. Filtersystem (2) gemäß Anspruch 2, **dadurch gekennzeichnet, dass** das separat ausgebildete Stellelement eine Schnappscheibe (32) ist.

4. Filtersystem (2') gemäß Anspruch 1, **dadurch gekennzeichnet, dass** der temperatursensitive Stellabschnitt (38) einstückig mit dem Filterelement (46) ausgebildet ist.

5. Filtersystem (2') gemäß Anspruch 4, **dadurch gekennzeichnet, dass** das Filterelement (46) insgesamt als eine Schnappscheibe (38) ausgebildet ist.

6. Filtersystem (2; 2') gemäß einem der vorstehenden Ansprüche, **dadurch gekennzeichnet, dass** das Filterelement (4; 46) durch mehrere gleichmäßig in der Umfangsrichtung verteilte, sich von einem Zentrum (40) zu einem umlaufenden Rand (42) erstreckende Stege (44) verstärkt ist.

7. Sterilbehälter (14) mit einem durch einen Sterilbehälterboden (12) und Sterilbehälterwänden (52) gebildeten Aufnahmeraum (54) und einem Sterilbehälterdeckel zum Verschließen des Aufnahmeraums (54), **dadurch gekennzeichnet, dass** mindestens ein Filtersystem (2; 2') nach einem der vorstehenden Ansprüche an einem Sterilbehälterwandungsabschnitt, welcher eine Gasaustauschöffnung (24) aufweist, befestigt ist.

8. Sterilbehälter (4) gemäß Anspruch 7, **dadurch gekennzeichnet, dass** das Filtersystem (2; 2') ferner eine Abdeckung (10) und eine als eine Art Blatt- oder Tellerfeder ausgebildete Feder (22) aufweist, welche eine Vorspannkraft entgegen der durch den temperatursensitiven Stellabschnitt (28; 38) aufgebrachten Kraft ausübt, und wobei die Vorspannkraft der Feder (22) kleiner als die durch den temperatursensitiven Stellabschnitt (28, 38) aufgebrachte Kraft ist, wenn die vorbestimmte Temperatur erreicht oder überschritten wird, und wobei die Vorspannkraft der Feder (22) größer als die durch den temperatursensitiven Stellabschnitt (28; 38) aufgebrachte Kraft ist, wenn die vorbestimmte Temperatur nicht erreicht oder überschritten wird.

9. Sterilbehälter (14) gemäß Anspruch 8, **dadurch gekennzeichnet, dass** sich die Feder (22) in der Mitte der Abdeckung (10) und/oder an dem Filterelement (4; 46) abstützt.

10. Sterilbehälter (14) gemäß einem der Ansprüche 8 oder 9, **dadurch gekennzeichnet, dass** in der Abdeckung (10) mindestens eine seitliche Öffnung (20) vorgesehen ist, wobei die seitliche Öffnung (20) auf der dem Sterilbehälterwandungsabschnitt zugewandten Seite der Abdeckung (10) vorgesehen ist, sodass ein in dem Aufnahmeraum (54) des Sterilbehälters (14) befindliches Fluid durch die seitliche Öffnung (20) und die Gasaustauschöffnung (24) aus dem Sterilbehälter (14) abgeführt werden kann.

11. Sterilbehälter (14) gemäß einem der Ansprüche 7 bis 10, **dadurch gekennzeichnet, dass** in dem Sterilbehälterboden (12) mehrere Öffnungen (26), welche die Gasaustauschöffnung (24) ausbilden, vorgesehen sind und wobei diese Öffnungen (26) vorzugsweise in einer Kreisform angeordnet sind und wobei besonders bevorzugt eine Kreissegment (62) in dem Sterilbehälterboden (12), an welchem sich der temperatursensitive Stellabschnitt (28; 38) abstützt, keine Öffnungen (26) aufweist.

12. Sterilbehälter (14) gemäß Anspruch 7, **dadurch gekennzeichnet, dass** das zumindest eine Filtersystem (2; 2') an einem Abschnitt des Sterilbehälterbodens (12) und/oder der Sterilbehälterwand (52) und/oder des Sterilbehälterdeckels befestigt ist.

## Claims

1. A gas-permeable filter system (2; 2') for a sterile container (14), comprising a filter element (4; 46), a seal portion (8) which is adapted to sealingly surround a gas exchange opening (24) provided in a sterile container wall section (12) in such a manner that the seal portion (8) rests against the sterile container wall section (12) of the sterile container (14), so that a sterile flow path is formed through the gas exchange opening (24) and the filter element (4; 46), and at least one temperature-sensitive adjusting section (28; 38) which compulsively releases at least the seal portion (8) from the sterile container wall section (12) when a specified temperature is reached or exceeded such that a non-sterile flow path is formed which allows fluid to be supplied or discharged via the gas exchange opening (24) while bypassing the filter element (4; 46),
**characterized in that**
the temperature-sensitive adjusting section (28; 38), when the specified temperature is reached or exceeded, moves the filter element (4; 46) from a first position in which the filter element (4; 46) is aligned parallel to the sterile container wall section (12) of the sterile container (14)to a second position in which the filter element (4; 46) is not aligned parallel to the sterile container wall section (12).

2. The filter system (2) according to claim 1, **characterized in that** the temperature-sensitive adjusting section (28) is at least one adjusting element formed separately from the filter element (4).

3. The filter system (2) according to claim 2, **characterized in that** the separately formed adjusting element is a snap disc (32).

4. The filter system (2') according to claim 1, **characterized in that** the temperature-sensitive adjusting section (38) is formed in one piece with the filter element (46).

5. The filter system (2') according to claim 2, **characterized in that** the filter element (46) as a whole is designed as a snap disc (38).

6. The filter system (2; 2') according to any of the preceding claims, **characterized in that** the filter element (4; 46) is reinforced by a plurality of uniformly circumferentially distributed webs (44) extending from a center (40) to a surrounding edge (42).

7. A sterile container (14) comprising a receiving space (54) made of a sterile container base (12) and sterile container walls (52) and comprising a sterile container cover for closing the receiving space (54), **characterized in that** at least one filter system (2; 2') according to any of the preceding claims is secured to the sterile container wall section which has a gas exchange opening (24).

8. The sterile container (4) according to claim 7, **characterized in that** the filter system (2; 2') further comprises a cover (10) and a spring (22) realized in the form of a leaf spring or disc spring, said spring exerting a preload force contrary to that of the temperature-sensitive adjusting section (28; 38), and wherein the preload force of the spring (22) is less than the force applied by the temperature-sensitive adjusting section (28, 38) when the specified temperature is reached or exceeded, and wherein the preload force of the spring (22) is greater than the force applied by the temperature-sensitive adjusting section (28; 38) when the specified temperature is not reached or exceeded.

9. The sterile container (14) according to claim 8, **characterized in that** the spring (22) is supported in the center of the cover (10) and/or on the filter element (4; 46).

10. The sterile container (14) according to any of claims 8 or 9, **characterized in that** at least one lateral opening (20) is provided in the cover (10), said lateral opening (20) being located on the side of the cover (10) facing the sterile container wall section so that a fluid located in the receiving space (54) of the sterile container (14) can be discharged from the sterile container (14) through the lateral opening (20) and the gas exchange opening (24).

11. The sterile container (14) according to any of claims 7 to 10, **characterized in that** several openings (26), which form the gas exchange opening (24), are provided in the sterile container base (12) and wherein these openings (26) are preferably arranged in a circular form and, particularly preferred, a circular segment (62) in the sterile container base (12), on which the temperature-sensitive adjusting section (28; 38) is supported, does not comprise any openings (26).

12. The sterile container (14) according to claim 7, **characterized in that** the at least one filter system (2; 2') is secured to a section of the sterile container base (12), and/or of the sterile container wall (52), and/or of the sterile container cover.

## Revendications

1. Système de filtration perméable aux gaz (2 ; 2') pour un récipient stérile (14), avec un élément de filtration (4 ; 46), une section d'étanchéité (8) qui est réalisée pour entourer de manière étanche une ouverture d'échange de gaz (24) prévue au niveau d'une section de paroi de récipient stérile (12) du récipient stérile (14) de manière adjacente à une section de paroi de récipient stérile (12) de sorte qu'un chemin d'écoulement stérile est réalisé à travers l'ouverture d'échange de gaz (24) et l'élément de filtration (4 ; 46) et au moins une section de réglage sensible à la température (28 ; 38) qui, lorsqu'une température prédéterminée est atteinte ou dépassée, détache de force au moins la section d'étanchéité (8) de la section de paroi de récipient stérile (12) de telle sorte qu'un chemin d'écoulement non stérile est réalisé, lequel permet d'amener ou d'évacuer un fluide tout en contournant l'élément de filtration (4 ; 46) par le biais de l'ouverture d'échange de gaz (24), **caractérisé en ce que**
la section de réglage sensible à la température (28 ; 38) lorsque la température prédéterminée est atteinte ou dépassée fait passer l'élément de filtration (4 ; 46) d'une première position dans laquelle l'élément de filtration (4 ; 46) est orienté parallèlement à la section de paroi de récipient stérile (12) du récipient stérile (14) à une seconde position dans laquelle l'élément de filtration (4 ; 46) n'est pas orienté parallèlement à la section de paroi de récipient stérile (12).

2. Système de filtration (2) selon la revendication 1, **caractérisé en ce que** la section de réglage sensible à la température (28) est au moins un élément de réglage réalisé séparément de l'élément de filtration (4).

3. Système de filtration (2) selon la revendication 2, **caractérisé en ce que** l'élément de réglage réalisé séparément est un disque à encliquetage (32).

4. Système de filtration (2') selon la revendication 1, **caractérisé en ce que** la section de réglage sensible à la température (38) est réalisée d'un seul tenant avec l'élément de filtration (46).

5. Système de filtration (2') selon la revendication 4, **caractérisé en ce que** l'élément de filtration (46) est réalisé dans l'ensemble en tant que disque à encliquetage (38).

6. Système de filtration (2 ; 2') selon l'une quelconque des revendications précédentes, **caractérisé en ce que** l'élément de filtration (4 ; 46) est renforcé par plusieurs nervures (44) réparties uniformément dans la direction périphérique et s'étendant d'un centre (40) vers un bord périphérique (42).

7. Récipient stérile (14) avec un espace de réception (54) formé par le biais d'un fond de récipient stérile (12) et des parois de récipient stérile (52) et un couvercle de récipient stérile destiné à la fermeture de l'espace de réception (54), **caractérisé en ce qu'**au moins un système de filtration (2 ; 2') selon l'une quelconque des revendications précédentes est fixé à une section de paroi de récipient stérile, laquelle présente une ouverture d'échange de gaz (24).

8. Récipient stérile (4) selon la revendication 7, **caractérisé en ce que** le système de filtration (2 ; 2') présente en outre un chapeau (10) et un ressort (22) réalisé en tant que ressort à lame ou ressort à disque, lequel exerce une force de précontrainte contre la force appliquée par le biais de la section de réglage sensible à la température (28 ; 38) et dans lequel la force de précontrainte du ressort (22) est plus petite que la force appliquée par le biais de la section de réglage sensible à la température (28, 38), lorsque la température prédéterminée est atteinte ou dépassée et dans lequel la force de précontrainte du ressort (22) est plus grande que la force appliquée par le biais de la section de réglage sensible à la température (28 ; 38) lorsque la température prédéterminée n'est pas atteinte ou dépassée.

9. Récipient stérile (14) selon la revendication 8, **caractérisé en ce que** le ressort (22) s'appuie au centre du chapeau (10) et/ou au niveau de l'élément de filtration (4 ; 46).

10. Récipient stérile (14) selon l'une quelconque des revendications 8 ou 9, **caractérisé en ce que** dans le chapeau (10) au moins une ouverture latérale (20) est prévue, dans lequel l'ouverture latérale (20) est prévue sur la face du chapeau (10) tournée vers la section de paroi de récipient stérile de sorte qu'un fluide qui se trouve dans l'espace de réception (54) du récipient stérile (14) peut être évacué du récipient stérile (14) par le biais de l'ouverture latérale (20) et de l'ouverture d'échange de gaz (24).

11. Récipient stérile (14) selon l'une quelconque des revendications 7 à 10, **caractérisé en ce que** dans le fond de récipient stérile (12) plusieurs ouvertures (26), lesquelles forment l'ouverture d'échange de gaz (24), sont prévues et dans lequel ces ouvertures (26) sont de préférence disposées en forme de cercle et dans lequel plus préférablement un segment de cercle (62) ne présente aucune ouverture (26) dans le fond de récipient stérile (12), au niveau duquel s'appuie la section de réglage sensible à la température (28 ; 38).

12. Récipient stérile (14) selon la revendication 7, **caractérisé en ce que** l'au moins un système de filtration (2 ; 2') est fixé à une section du fond de récipient stérile (12) et/ou de la paroi de récipient stérile (52) et/ou du couvercle de récipient stérile.
